# EUROPÄISCHE PATENTSCHRIFT

(11) **EP 0 457 370 B1**
(45) Veröffentlichungstag und Bekanntmachung des Hinweises auf die Patenterteilung: **03.05.1995**
(21) Anmeldenummer: 91112381.8
(22) Anmeldetag: 15.12.1987
(51) Int. Cl.: A61K 35/50, A61K 38/02, A61L 2/00

(54) **Verfahren zur Pasteurisierung des Gewebeproteins PP4**
Method for the pasteurization of the tissue protein PP4
Procédé pour la pasteurisation de la protéine tissulaire PP4

(30) Priorität: 18.12.1986 DE 3643182
(43) Veröffentlichungstag der Anmeldung: 21.11.1991
(62) Teilanmeldung aus: 87118598.9
(73) Patentinhaber: BEHRINGWERKE Aktiengesellschaft, 35001 Marburg (DE)
(72) Erfinder: Löbermann, Hartmut Dr., W-7801 Schallstadt (DE); Bornmann, Chritiane, W-3573 Gemünden (Wohra) (DE)

(56) Entgegenhaltungen:
- EP-A- 317 341
- EP-A- 0 106 269
- EP-A- 0 123 307
- EUROPEAN JOURNAL OF BIOCHEMISTRY, Band 151, Nr. 3, 16. September 1985, Seiten625-629, FEBS; C.P.M. REUTELINGSPERGER et al.: "Isolation and partialpurification of a novel anticoagulant from arteries of human umbilical cord"

## Beschreibung

Die Erfindung betrifft ein Verfahren zur Pasteurisierung des Gewebeproteins PP4.

In DE 33 15 000 A 1 (USP 4,507,229) ist das Glycoprotein PP4 beschrieben, das beispielsweise in Placenta gefunden wird. PP4 besitzt ein Molekulargewicht von 35 000 ± 5 000 Dalton und einen isoelektrischen Bereich von pH 4.85 ± 0.15.

Blutgerinnungshemmende Proteine lassen sich aufgrund ihrer Wirkungsweise in mehrere Gruppen einteilen. Sowohl die Gruppe der Proteinase-Inhibitoren, wie beispielsweise Antithrombin III, als auch der Proteinasen, beispielsweise aktiviertes Protein C, bringen zum Teil nachteilige Eigenschaften für eine gerinnungshemmende Therapie mit sich, da sie entweder erst auf der Stufe der aktivierten Gerinnungsenzyme wirken oder aber durch eine Proteolyse von Gerinnungsfaktoren diese desaktivieren, sie "verbrauchen".

Gegenstand der Stammanmeldung zu dieser Teilanmeldung ist ein die Blutgerinnung hemmendes Mittel, das die Blutgerinnung verhindern kann, ohne zu einem Verbrauch von Gerinnungsfaktoren zu führen.

In DE 35 33 516 A 1, Seiten 6 bis 8, sind blutgerinnungshemmende Proteine beschrieben, für die ein isoelektrischer Bereich von 4.4 - 4.6 angegeben ist.

Diese Proteine werden aus der Intimaschicht größerer Gefäße, beispielsweise Aorta oder Nabelschnur, isoliert.

In Acta Obst. Gynaec. Jpn. 36, No. 12, 2583-2592 (1984) ist ebenfalls ein gerinnungshemmendes Protein aus Placenta beschrieben, das ein Molekulargewicht von 45 000 Dalton hat. In DE 33 15 000 A 1 (USP 4,507,229) sind auch die folgenden Eigenschaften von PP4 beschrieben:
eine elektrophoretische Beweglichkeit im Bereich zwischen der von alpha₁ und alpha₂ Globulinen; ein Sedimentationskoeffizient 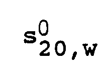
von 3,3 ± 0,2 S; ein Extinktionskoeffizient 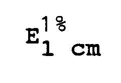
(280 nm) von 5,9 ± 0,6; ein Kohlenhydratanteil von 2,4 ± 0,94 % (g/100 g) (Mannose 0,3 ± 0,2 %, Galactose 0,4 ± 0,2 %, Xylose 0,1 % ± 0,04 %, Glukose 0,2 ± 0,1 %, Glukosamin 1,0 + 2 %, Neuraminsäure 0,4 ± 0,2 %) und die folgenden Aminosäurenzusammensetzung:

| **Aminosäure** | **Reste pro 100 Reste (mol %)** | **Variationskoeffizient** |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

Wegen der Gefahr einer Übertragung von Krankheiten (Hepatitis, AIDS) ist es zweckmäßig für eine Anwendung am Menschen bestimmte biologische Therapeutika zur Abtötung infektiöser Keime zu erhitzen. Dies bedeutet jedoch meist Aktivitätsverlust der biologischen Wirkstoffe.

Es wurde überraschenderweise gefunden, daß eine wässrige Lösung von PP4 in Gegenwart von Calciumionen sowie mindestens eines Mono- oder Oligosaccharides oder Zuckeralkohols und gegebenenfalls einer Aminosäure erhitzt werden kann, wobei die blutgerinnungshemmende Eigenschaft weitestgehend erhalten bleibt.

Gegenstand der Erfindung ist deshalb ein Verfahren zur Pasteurisierung einer PP4 enthaltenden Lösung, dadurch gekennzeichnet, daß diese Lösung in Gegenwart von Calciumionen sowie mindestens eines Mono- oder Oligosaccharids oder Zuckeralkohols und gegebenenfalls einer Aminosäure unter Bedingungen so erhitzt wird, daß pathogene Keime abgetötet werden.

In einer bevorzugten Verfahrensweise zur Pasteurisierung einer PP4 enthaltenden Lösung wird der pH-Wert der Lösung auf 5.0 - 9.5 eingestellt und 30 - 150 g/100 ml Lösung mindestens eines Mono- oder Oligosaccharides oder Zuckeralkohols und 0.005 - 2 mol/l Calciumionen sowie gegebenenfalls 0.1 - 1.5 mol/l einer Aminosäure zugesetzt. Die Calciumionen können beispielsweise in Form von Calciumsalzen, beispielsweise als CaCl₂ oder Calciumacetat zugeführt werden.

In einer ganz besonders bevorzugten Ausführungsform wird eine PP4 enthaltende Lösung, die einen pH-Wert von 6.5 - 8.5 besitzt, mit 100 - 150 g Saccharose/100 ml Lösung und 30 - 100 mmol/l CaCl₂ versetzt. Nach dem Lösen der Bestandteile kann die Lösung beispielsweise 1 bis 20 Stunden bei 40 - 80°C, vorzugsweise aber 8 - 15 Stunden bei 55 - 65°C erhitzt werden. Der Gehalt an PP4 sollte nicht 20 »g/ml Lösung unterschreiten. Nach der Erhitzung kann die Lösung beispielsweise durch Konzentrierung, Dialyse, Sterilfiltration und/oder Lyophilisation weiterverarbeitet werden.

Auch ein gentechnisch hergestelltes PP4 kann im erfindungsgemäßen Sinn verwendet werden.

Ein nach diesem Pasteurisierungsverfahren erhaltenes Präparat zeichnet sich besonders dadurch aus, daß die blutgerinnungshemmende Wirkung weitestgehend vorhanden ist.

Die Erfindung soll durch das nachstehende Beispiel erläutert werden.

### Beispiel

### Pasteurisierung einer PP4 enthaltenden Lösung:

500 ml einer PP4 enthaltenden Lösung (100 »g pp4/ml) wurden gegen 20 mmol/Tris/HCl, 150 mmol NaCl, pH 7,5 dialysiert. Anschließend wurden 100 g Saccharose/100 ml Lösung zugewogen. Nach dem vollständigen Lösen der Saccharose wurden 50 mmol/l CaCl₂ zugesetzt. Nach dem Erhitzen (10 h/60°C) kann die Lösung gegen 20 mmol/l Tri-Na-Citrat, 60 mmol/l NaCl, 10 g/l Glycin, pH 7.0 dialysiert, sterilfiltriert und lyophilisert werden.

### Bestimmung der blutgerinnungshemmenden Aktivität von PP4:

Zur Bestimmung der blutgerinnungshemmenden Aktivität von PP4 wurde die modifizierte Prothrombinzeit (PT)-Bestimmung verwendet.

### Modifizierte PT-Bestimmung

Zu 50 »l Standard-Human-Plasma wurden 150 »l Puffer A (20 mmol/l Tris/HCl, 142 mmol NaCl pH 7.5), 25 »l Probe oder Puffer B (20 mmol/l Tris/HCl, 500 mmol/l NaCl pH 7.5) und 25 »l Thromboplastinlösung zugesetzt. Nach einer dreiminütigen Inkubation bei 37°C wurde die Gerinnung durch Zugabe von 250 »l einer CaCl₂ enthaltenden Lösung C (10 mmol/l Tris/HCl, 80 mmol/l NaCl, 20 mmol/l CaCl₂ pH 7.9) gestartet und die Gerinnungszeit in einem Schnitger-Gross-Koagulometer bestimmt. Die Gerinnungszeit der Kontrolle mit Puffer B betrug 60 sec.

### Abhängigkeit der Gerinnungszeit in der modifizierten PT-Bestimmung von dem Gehalt an PP4 in der Lösung

| | | | | | |
|---|---|---|---|---|---|
| PP4- Konzentrat (»g/ml) | 0 | 5 | 10 | 15 | 20 |
| modifiz. PT-Zeit (sec) | 60 | 68 | 89 | 111 | 132.5 |

Pasteurisierung von PP4 (100 »g/ml) in Lösung - Einfluß von Calcium-Ionen auf die Stabilität von PP4:

| Modifizierte PT-Zeit (sec) | | |
|---|---|---|
| vor Erhitzung | Erhitzung ohne Calcium | Erhitzung mit 50 mmol/l Calcium |
| 68 | 60 | 68 |

## Patentansprüche

1. Verfahren zur Pasteurisierung einer das Gewebeprotein PP4 enthaltenden Lösung, dadurch gekennzeichnet, daß diese Lösung in Gegenwart von Calciumionen sowie mindestens eines Mono- oder Oligosaccharids oder Zuckeralkohols und gegebenenfalls einer Aminosäure unter Bedingungen erhitzt wird, daß pathogene Keime abgetötet werden.

2. Verfahren nach Anspruch 1, dadurch gekennzeichnet, daß bei einem pH-Wert von 5.0 - 9.5, vorzugsweise 6.5 - 8.5 erhitzt wird.

3. Verfahren nach Anspruch 1, dadurch gekennzeichnet, daß 30 - 150, vorzugsweise 100 - 150 g/100 ml Lösung eines Mono- oder Oligosaccharids oder Zuckeralkohols zugesetzt werden.

4. Verfahren nach Anspruch 1, dadurch gekennzeichnet, daß 100 - 150 g Saccharose/100 ml Lösung zugesetzt werden.

5. Verfahren nach Anspruch 1, dadurch gekennzeichnet, daß 0.005 - 2 mol/l, vorzugsweise 30 - 100 mmol/l Calciumionen zugesetzt werden.

6. Verfahren nach Anspruch 1, dadurch gekennzeichnet, daß 0.1 - 1.5 mol/l einer Aminosäure zugesetzt werden.

## Claims

1. A process for the pasteurization of a solution containing the tissue protein PP4, wherein this solution is heated in the presence of calcium ions and also of at least one monosaccharide or oligosaccharide or sugar alcohol and, where appropriate, of an amino acid, under conditions such that pathogenic microorganisms are destroyed.

2. The process as claimed in claim 1, wherein the heating is carried out at a pH of 5.0 - 9.5, preferably 6.5 - 8.5.

3. The process as claimed in claim 1, wherein 30 - 150, preferably 100 - 150, g of a monosaccharide or oligosaccharide or sugar alcohol are added per 100 ml of solution.

4. The process as claimed in claim 1, wherein 100 - 150 g of sucrose are added per 100 ml of solution.

5. The process as claimed in claim 1, wherein 0.005 - 2 mol, preferably 30 - 100 mmol, of calcium ions are added per liter.

6. The process as claimed in claim 1, wherein 0.1 - 1.5 mol of an amino acid are added per liter.

## Revendications

1. Procédé pour la pasteurisation d'une solution contenant la protéine tissulaire PP4, caractérisé en ce que l'on chauffe cette solution en présence d'ions calcium ainsi que d'au moins un mono- ou oligosaccharide ou alcool glucidique, et éventuellement d'un aminoacide, dans des conditions telles que les germes pathogènes sont tués.

2. Procédé selon la revendication 1, caractérisé en ce que le chauffage est effectué à un pH de 5,0-9,5, de préférence de 6,5-8,5.

3. Procédé selon la revendication 1, caractérisé en ce que l'on ajoute 30-150, de préférence 100-150 g d'un mono- ou oligosaccharide ou alcool glucidique pour 100 ml de solution.

4. Procédé selon la revendication 1, caractérisé en ce que l'on ajoute 100-150 g de saccharose pour 100 ml de solution.

5. Procédé selon la revendication 1, caractérisé en ce que l'on ajoute 0,005-2 moles/l, de préférence 30-100 mmoles/l, d'ions calcium.

6. Procédé selon la revendication 1, caractérisé en ce que l'on ajoute 0,1-1,5 mole/l d'un aminoacide.
